# EUROPEAN PATENT SPECIFICATION

(11) **EP 3 153 014 B1**
(45) Date of publication and mention of the grant of the patent: **30.12.2020**
(21) Application number: 15803135.1
(22) Date of filing: 28.05.2015
(51) Int. Cl.: A01H 4/00, A01H 5/00, A01N 39/04, A01N 43/60, A01P 21/00, C12N 5/00, C12N 15/82

(54) **AGENT FOR INDUCING PLANT CALLUS AND METHOD FOR INDUCING PLANT CALLUS**
MITTEL UND VERFAHREN FÜR INDUKTION VON PFLANZEN-KALLUS
AGENT ET MÉTHODE D'INDUCTION DE CALS VÉGÉTAUX

(30) Priority: 06.06.2014 JP 2014117832
(43) Date of publication of application: 12.04.2017
(73) Proprietor: Riken, Wako-shi, Saitama 351-0198 (JP)
(72) Inventor: NAKANO Takeshi, Wako-shi Saitama 351-0198 (JP); ASAMI Tadao, Wako-shi Saitama 351-0198 (JP); YAMAGAMI Ayumi, Wako-shi Saitama 351-0198 (JP); OSADA Hiroyuki, Wako-shi Saitama 351-0198 (JP); OHTANI Misato, Wako-shi Saitama 351-0198 (JP); DEMURA Taku, Wako-shi Saitama 351-0198 (JP)
(74) Representative: J A Kemp LLP
(86) International application number: PCT/JP2015/065332
(87) International publication number: WO 2015/186591

(56) References cited:
- EP-A1- 2 679 241
- EP-A2- 0 090 203
- WO-A2-98/56771
- JP-A- S62 201 572
- JP-A- S62 201 572
- JP-A- 2002 503 239
- JP-A- 2003 047 463
- JP-A- 2003 047 463
- TANAKA SHOTA ET AL: "Physiological function of FPX as a novel plant callus inducible compound and PPG as a novel promoter for plant growth", SHOKUBUTSU-NO-SEICHÔ-CHÔSETSU = REGULATION OF PLANT GROWTH AND DEVELOPMENT, TOKYO : SHOKUBUTSU KAGAKU CHÔSETSU GAKKAI, vol. 49, no. Suppl, 1 October 2014 (2014-10-01), page 58, XP008185340, ISSN: 1346-5406
- SHOTA TANAKA ET AL: "2P11(0727): Shinki Shokubutsu Callus Keisei Sokushin Kagobutsu FPX to Shinki Shokubutsu Seicho Sokushin Kagobutsu PPG no Dotei to Seiri Sayo Kaiseki", DAI 56 KAI PROCEEDINGS OF THE ANNUAL MEETING OF THE JAPANESE SOCIETY OF PLANT PHYSIOLOGISTS, JAPANESE SOCIETY OF PLANT PHYSIOLOGISTS, JP, vol. 56, no. Suppl, 9 March 2015 (2015-03-09), page 307, XP008185420,
- SHOTA TANAKA ET AL.: 'Physiological function of FPX as a novel plant callus inducible compound and PPG as a novel promoter for plant growth' JAPANESE SOCIETY FOR CHEMICAL REGULATION OF PLANTS DAI 49 KAI TAIKAI KENKYU HAPPYO KIROKUSHU vol. 49, 01 October 2014, page 58, XP008185340
- SHOTA TANAKA ET AL.: 'Shinki Shokubutsu Callus Keisei Sokushin Kagobutsu FPX to Shinki Shokubutsu Seicho Sokushin Kagobutsu PPG no Dotei to Seiri Sayo Kaiseki' DAI 56 KAI PROCEEDINGS OF THE ANNUAL MEETING OF THE JAPANESE SOCIETY OF PLANT PHYSIOLOGISTS 09 March 2015, page 307, 2P11, XP008185420
- TAKESHI NAKANO ET AL: "FPX is a Novel Chemical Inducer that Promotes Callus Formation and Shoot Regeneration in Plants", PLANT AND CELL PHYSIOLOGY, vol. 59, no. 8, 20 July 2018 (2018-07-20), pages 1555-1567, XP055498668, UK ISSN: 0032-0781, DOI: 10.1093/pcp/pcy139
- LEKHA SLENO ET AL: "Investigating thein vitro metabolism of fipexide: characterization of reactive metabolites using liquid chromatography/mass spectrometry", RAPID COMMUNICATIONS IN MASS SPECTROMETRY., vol. 21, no. 14, 1 January 2007 (2007-01-01), pages 2301-2311, XP055606925, GB ISSN: 0951-4198, DOI: 10.1002/rcm.3092
- Huang Tao ET AL: "Plant regeneration from leaf-derived callus in Citrus grandis(pummelo): Effects of auxins in callus induction medium", Plant Cell, Tissue and Organ Culture, 1 May 2002 (2002-05-01), pages 141-146, XP055606905, Dordrecht DOI: 10.1023/A:1015223701161 Retrieved from the Internet: URL:https://link.springer.com/content/pdf/ 10.1023/A:1015223701161.pdf
- Anonymous: "Effects of 4-Chlorophenoxyacetic Acid on the Callus and Adventitious Organogenesis from Cultured Leaf Explant African Violet In Vitro. | Article Information | J-GLOBAL", , 22 April 2009 (2009-04-22), XP055606916, Retrieved from the Internet: URL:https://jglobal.jst.go.jp/en/detail?JG LOBAL_ID=200902194559794385&rel=0 [retrieved on 2019-07-18]
- Elisa Ferreira Moura ET AL: "INDUCTION OF SOMATIC EMBRYOGENESIS IN IMMATURE SEEDS OF GUAVA TREE cv. PALUMA 1 INTRODUCTION", Rev. Bras. Frutic, 1 June 2009 (2009-06-01), pages 507-511, XP055606929, Retrieved from the Internet: URL:http://www.scielo.br/pdf/rbf/v31n2/v31 n2a27.pdf [retrieved on 2019-07-18]
- Roy U. Schenk ET AL: "Medium and techniques for induction and growth of monocotyledonous and dicotyledonous plant cell cultures", CANADIAN JOURNAL OF BOTANY / JOURNAL CANADIEN DE BOTANIQUE, vol. 50, no. 1, 1 January 1972 (1972-01-01), pages 199-204, XP055663469, CA ISSN: 0008-4026, DOI: 10.1139/b72-026
- Peter R. Rich ET AL: "Cytochrome Components of Plant Microsomes", European Journal of Biochemistry, vol. 55, no. 2, 1 July 1975 (1975-07-01), pages 333-341, XP055663535, GB ISSN: 0014-2956, DOI: 10.1111/j.1432-1033.1975.tb02167.x

## Description

### Technical Field

The present specification relates to an agent for inducing a plant callus and a method for inducing a plant callus.

### Background Art

Plants have totipotency, such that they are able to form calluses from somatic cells that have been highly differentiated. If calluses are cultured under constant conditions, also, plant bodies can be reproduced through differentiation of adventitious embryos, adventitious buds, and adventitious roots. Callus culture is advantageous in the following respect; for example, 1) it has reproductive integrity; 2) it has the ability to differentiate a callus cell mass into various tissues and individuals; 3) a large quantity of homogeneous growth cells that are difficult to obtain in plants can be obtained; 4) a callus is suitable as an experimental material because of the absence of seasonal changes in the quality or quantity of cells; 5) the influence of a substance that has been added to the medium on plants can be directly observed; and 6) some plant species are easily induced to undergo genetic variation as a result of callus formation, and such plants can be used for breeding. Thus, callus culture has been extensively used for the production of useful materials, the development of new varieties, the gene introduction into plants, the reproduction of transformants, the production of artificial seeds, and other purposes.

In general, a callus is produced by a method in which a piece of plant tissue is cultured in a medium containing phytohormones (i.e., auxin and cytokinine) (Skoog, F., and Miller, C.O., 1957, Chemical regulation of growth and organ formation in plant tissue cultured in vitro, Symp. Soc. Exp. Biol., 54, 118-130). This method was established over 30 years ago, and this technique is indispensable for the production of transgenic crops at present. However, types of auxin and cytokinine to be used and the amount of auxin relative to that of cytokinine vary depending on plant species. Accordingly, it has been difficult to find adequate conditions for callus formation.

As synthetic auxins, 2,4-dichlorophenoxyacetic acid and 2,4,5-trichlorophenoxy acetic acid are known, and, concerning an analog thereof; i.e., 4-chlorophenoxyacetic acid, it was reported that the number of days required for leaf tissue culture of African violet, from implanting of explants to callus formation, and formation of adventitious shoots and adventitious roots, was reduced as a result of treatment performed at a particular concentration (Miyoshi Hakozaki et al., Effects of 4-Chlorophenoxyacetic Acid on the Callus and Adventitious Organogenesis from Cultured Leaf Explant African Violet in Vitro, Memoirs of the Institute of Science and Technology, Meiji University, 40, 1-7, 2001). However, callus formation efficiency was not high, and plant species capable of callus formation were limited.

As a 1,4-disubstituted piperazine derivative, JP H6-67668 (B) (1994) describes that N-methyl-N'-phenylacetylpiperazine, N-benzyl-N'-benzoylpiperazine, and the like are useful as decolorants against the color development system of bicolor thermal recording materials.

JP 2002-503239 (A) describes that a piperazine derivative having substituents at positions other than 1- and 4-positions of the piperazine ring is useful as an anti-inflammatory agent.

Fipexide, which is a type of a 1,4-disubstituted piperazine derivative (e.g., 1-[(p-chlorophenoxy)acetyl]-4-piperonylpiperazine or 1-(4-chlorophenoxyacetyl)-4-(1,3-benzodioxole-5-ylmethyl)piperazine), is known as an antidepressant.

Further, 1-piperonylpiperazine (1-(3,4-methylenedioxybenzyl)piperazine) and many 1-substituted piperazine derivatives as analogs thereof are commercially available as raw materials for chemical production.

However, there has been no report demonstrating that 1,4-disubstituted piperazine derivatives and 1-substituted piperazine derivatives are capable of callus induction.

### Summary

### Objects to Be Attained by the Specification

It is an object of the present specification to provide an agent for inducing a plant callus, which has a basic structure different from that of a conventional agent for inducing a plant callus, and a method for inducing a plant callus using such agent for inducing a plant callus.

### Means for Attaining the Objects

The present invention is summarized as follows.
(1) Use of an agent for inducing a plant callus wherein the agent comprises a compound represented by Formula (I) or a salt thereof: 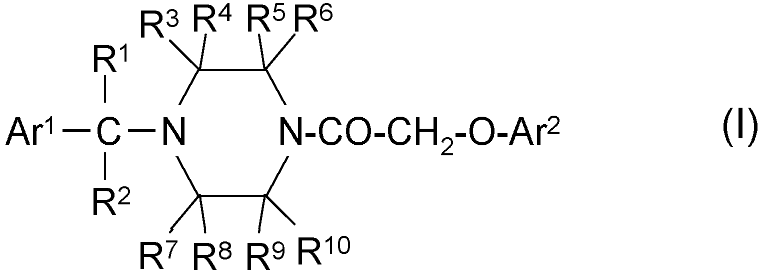
   wherein Ar¹ represents a 3,4-methylenedioxyphenyl group;
   Ar² represents a 4-chlorophenyl group;
   R¹ and R² each represent a hydrogen atom; and
   R³, R⁴, R⁵, R⁶, R⁷, R⁸, R⁹, and R¹⁰ each represent a hydrogen atom.
(2) Use according to item (1), wherein the compound represented by Formula (I) or a salt thereof is fipexide.
(3) Use of an agent for inducing a plant callus wherein the agent comprises a compound represented by Formula (I-1) or a salt thereof: 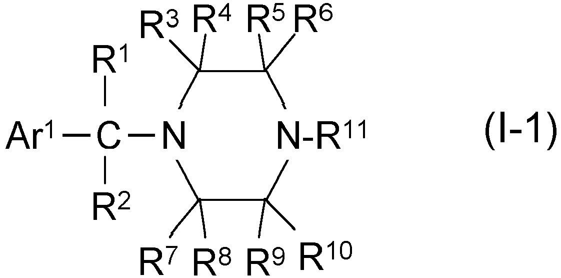 wherein Ar¹, R¹, R², R³, R⁴, R⁵, R⁶, R⁷, R⁸, R⁹, and R¹⁰ are as defined above with respect to Formula (I) in item (1); and R¹¹ represents a hydrogen atom, and a compound represented by Formula (I-2) or a salt thereof:

   HO-CO-CH₂-O-Ar² (I-2)

   wherein Ar² is as defined above with respect to Formula (I) in item (1).
(4) Use according to item (3), wherein the compound represented by Formula (I-1) or a salt thereof is 1-piperonylpiperazine and the compound represented by Formula (1-2) or a salt thereof is 4-chlorophenoxyacetic acid.
(5) A method for inducing a plant callus comprising bringing a plant, a plant cell, a piece of plant tissue, or a plant seed into contact with the agent as defined in any one of items (1) to (4) and inducing callus formation.
(6) A method for producing a plant callus comprising bringing a plant, a plant cell, a piece of plant tissue, or a plant seed into contact with the agent as defined in any one of items (1) to (4), inducing callus formation, and growing the callus.
(7) A method according to item (6), which further involves using the plant callus to produce a transformant.

### Effects Described Herein

The agent for inducing a plant callus according to the present specification comprises, as an active ingredient, a 1,4-disubstituted piperazine derivative or a 1-substituted piperazine derivative having a basic structure different from a conventional agent for inducing a plant callus.

Thus, such agent can be applied to a wide variety of plants.

### Brief Description of the Drawings

Fig. 1 shows the results of the callus induction activity test of fipexide (FPX) using *Arabidopsis.*
Fig. 2 shows the redifferentiation conditions as a result of treatment with fipexide (A) and without treatment (B).
Fig. 3 shows the results of a comparison of callus induction efficiency attained with the aid of fipexide (FPX) and that attained via a conventional technique (with the aid of 2,4-dichlorophenoxyacetic acid (2,4-D)/ kinetin).
Fig. 4 shows the results of the callus induction activity tests of fipexide (FPX), 4-chlorophenoxyacetic acid (CPA) alone, 1-piperonylpiperazine (PPZ) alone, and 4-chlorophenoxyacetic acid (CPA) in combination with 1-piperonylpiperazine (PPZ).
Fig. 5 shows the results of the callus induction activity test of fipexide (FPX) using the wild-type rice (Nipponbare).
Fig. 6 shows the results of the callus induction activity test of fipexide (FPX) on soybean seed (Tsurunoko), tomato seed (Micro-Tom), and cucumber seed (Natsu Suzumi).
Fig. 7 shows the results of the callus induction activity test of fipexide (FPX) using wild-type *Populus.*
Fig. 8 shows the results of plant transformation performed by the *Agrobacterium* method that employs callus induction caused by fipexide.

### Aspects for Implementing the Specification

Hereafter, the present specification is described in detail. The present specification relates to the use of an agent for inducing a plant callus and a method for inducing a plant callus with the use of a compound comprising a particular type of substituted piperazine skeleton in which a nitrogen atom is substituted with Ar¹-C(R¹)(R²)- and a phenoxyacetyl group (-CO-CH₂-O-Ar²) as defined herein or with the use of two types of compounds comprising the substituted piperazine skeleton and the substituted phenoxyacetyl group in separate molecules in combination.

The correlation between the compound comprising the substituted piperazine skeleton and plant callus induction has not yet been reported.

The compounds comprising a phenoxyacetyl group (-CO-CH₂-O-Ar²), this being 4-chlorophenoxyacetic acid, are known as synthetic auxins.

Ar¹ is a 3,4-methylenedioxyphenyl group (a 1,3-benzodioxol-5-yl group).

Ar² in Formulae (I) and (1-2) is a 4-chlorophenyl group.

R¹ and R² are hydrogen atoms.

In Formulae (I) and (I-1), R³, R⁴, R⁵, R⁶, R⁷, R⁸, R⁹, and R¹⁰ are hydrogen atoms.

Among the compounds represented by Formula (I), fipexide in which a substituted phenyl group represented by Ar¹ is a 3,4-methylenedioxyphenyl group (a 1,3-benzodioxol-5-yl group), a substituted phenyl group represented by Ar² is a 4-chlorophenyl group, and R¹, R², R³, R⁴, R⁵, R⁶, R⁷, R⁸, R⁹, and R¹⁰ are each a hydrogen atom is a commercially available compound known as an antidepressant comprising a small number of substituents. This compound is preferable from the viewpoint of availability.

Among the compounds represented by Formula (I-1), 1-piperonylpiperazine in which a substituted phenyl group represented by Ar¹ is a 3,4-methylenedioxyphenyl group (a 1,3-benzodioxol-5-yl group) and R¹, R², R³, R⁴, R⁵, R⁶, R⁷, R⁸, R⁹, R¹⁰, and R¹¹ are each a hydrogen atom is a commercially available compound comprising a small number of substituents. This compound is preferable from the viewpoint of availability.

Examples of salts of the compounds represented by Formula (I) or (I-1) include salts with inorganic acids, such as hydrochloric acid, sulfuric acid, phosphoric acid, hydrobromic acid, hydroiodic acid, nitric acid, pyrosulfuric acid, and metaphosphoric acid, and salts with organic acids, such as citric acid, benzoic acid, acetic acid, propionic acid, fumaric acid, maleic acid, tartaric acid, succinic acid, sulfonic acid (e.g., methanesulfonic acid, p-toluenesulfonic acid, or naphthalenesulfonic acid), and amino acid (e.g., glutamic acid).

Examples of salts of the compounds represented by Formula (1-2) include alkaline metal salts, such as sodium salt and potassium salt, lysine salt, and arginine salt.

The compounds represented by Formula (I) can be produced in accordance with a conventional technique, such as the method described in JP 2002-503239 A, in the manner described below: 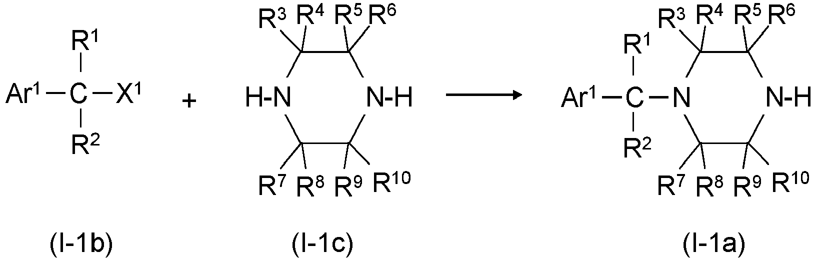 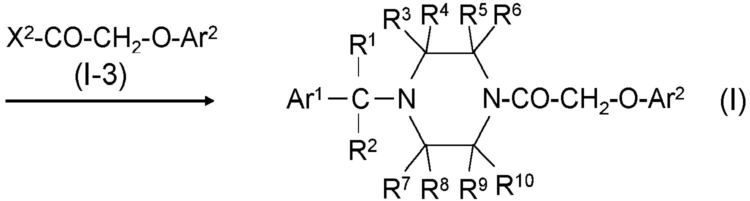 wherein Ar¹, Ar², R¹, R², R³, R⁴, R⁵, R⁶, R⁷, R⁸, R⁹, and R¹⁰ are as defined above; and X¹ and X² each independently represent a halogen atom (a chlorine, bromine, or iodine atom).

A compound represented by Formula (I-1a) is equivalent to a compound represented by Formula (I-1), wherein R¹¹ represents a hydrogen atom.

Products that can be obtained in the manner described above may be purified in accordance with a conventional technique, such as column chromatography using silica gel as a carrier, or recrystallization using methanol, ethanol, chloroform, dimethylsulfoxide, or water. Examples of eluting solvents used for column chromatography include methanol, ethanol, chloroform, acetone, hexane, dichloromethane, ethyl acetate, and a solvent mixture of any thereof.

Many of the compounds represented by Formulae (I), (I-1), and (I-2) are commercially available, and such commercially available compounds can be used.

Examples of plants to which the present specification is applicable include: dicotyledonous plants, such as *Arabidopsis* plants, trees (e.g., *Populus* and *Eucalyptus*), rapeseed, tomato, tobacco, soybean, carrot, melon, apple, *Manihot utilissima, Spirodela polyrhiza,* and *Striga;* and monocotyledonous plants, such as *Gramineae* plants (e.g., rice, wheat, barley, maize, and *Brachypodium distachyon*) and *Liliaceae* plants (e.g., onion).

Plants, plant cells, pieces of plant tissue, or plant seeds to be implanted into media provided that they are capable of callus induction. Examples of plant tissues include shoot apex, stalk, leaf, shoot, germ cell, and root. Plants and the like to be implanted are preferably disinfected and sterilized with the use of, for example, an aqueous solution of sodium hypochlorite. Plants and the like grown by aseptic seeding are not necessarily sterilized.

The agent for inducing a plant callus used herein can contain known additives for preparation, in addition to the active ingredients described above. Examples of known additives for preparation include an excipient, an emulsifier, and a moistening agent. The configuration of the agent for inducing a plant callus include an emulsion, a liquid, an oil solution, an aqueous solution, water dispersible powder, a flowable agent, a dusting powder, a microgranule, a granule, an aerosol, and a paste.

In the method for inducing a plant callus and the method for producing a plant callus plants and the like can be brought into contact with the agent for inducing a plant callus used herein. In accordance with a plant type, a target organ, a dosage form of the agent for inducing a plant callus, and other factors, an adequate means can be selected from among soaking, coating, spraying, and addition to the medium. To this end, plants and the like are preferably cultured in a medium containing the agent for inducing a plant callus.

Any plant callus induction medium may be used provided that it contains (i) the compound represented by Formula (I) or a salt thereof or (ii) the compound represented by Formula (I-1) or a salt thereof and the compound represented by Formula (I-2) or a salt thereof and it is capable of plant callus induction.

The concentration of the compound represented by Formula (I) or a salt thereof in the medium is generally 5 to 200 µM and preferably 15 to 60 µM.

When the compound represented by Formula (I-1) or a salt thereof is used in combination with the compound represented by Formula (I-2) or a salt thereof, the concentration of the compound represented by Formula (I-1) or a salt thereof in the medium is generally 0.01 to 100 µM, and preferably 0.1 to 60 µM. While the concentration of the compound represented by Formula (1-2) or a salt thereof in the medium is generally 0.01 to 100 µM, and preferably 0.1 to 60 µM.

Components other than the compound described above may be, for example, a saccharide, a gelling agent, or an inorganic salt, which is generally used for plant callus induction. Phytohormone may be added to the medium, provided that the effects of the present specification are not adversely affected.

Culture is preferably conducted under aseptic conditions. Culture is preferably carried out at 20°C to 25°C, and light conditions are preferably set between continuous light conditions and continuous dark conditions. In general, plant callus induction is observed 2 to 4 weeks after the initiation of culture.

In order to grow the plant callus that was induced in the manner described above, for example, the medium may be exchanged with a fresh callus induction medium (i.e., agarose solid MS medium or liquid MS medium containing 0.9% sucrose) every month.

The grown plant callus may be redifferentiated by, for example, transferring the plant callus to a MS medium (0.9% agarose and 1.5% sucrose) containing auxin (indoleacetic acid) at 0.15 mg/l and cytokinine (N⁶-2-isopentenyladenine) at 0.5 mg/l and developing a shoot (an adventitious bud) about 2 to 4 weeks thereafter.

The plant callus obtained according to the present specification can be used for production of a transgenic crop. For example, a plant callus may be prepared from a cell infected with *Agrobacterium,* or a callus may be infected with *Agrobacterium* comprising a plasmid to be introduced into a plant callus cell. Thereafter, a cell comprising a plasmid inserted into the chromosome may be separated and then redifferentiated to reproduce a plant. Thus, a transgenic crop that is capable of stable gene transfer can be obtained.

Japanese Patent Application No. 2014-117832, is a priority document of the present application.

### Examples

Hereafter, the present specification is described in greater detail with reference to the examples.

### [Example 1] Fipexide activity test using Arabidopsis

### (1) Morphological observation of Arabidopsis (Columbia) by treatment with fipexide

Wild-type seeds of *Arabidopsis (Columbia)* were sowed in a 1/2 MS medium supplemented with fipexide at 0, 15, 30, or 45 µM, and morphological observation was conducted.

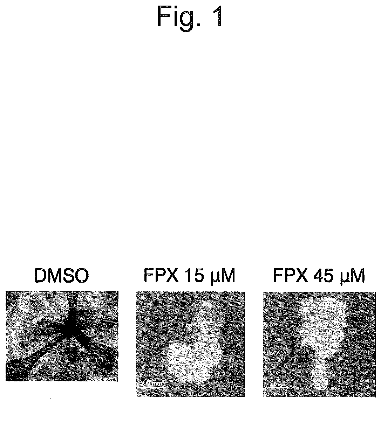

As a result of treatment with fipexide, callus formation was mainly observed in the root at low concentration of 15 µM and in the shoot apex at high concentration of 45 µM (Fig. 1). Since callus formation is suppressed in the root at high concentration, it was considered that the root was more sensitive to fipexide than the shoot apex and cell division was inhibited in the root.

### (2) Callus induction and redifferentiation by treatment with fipexide

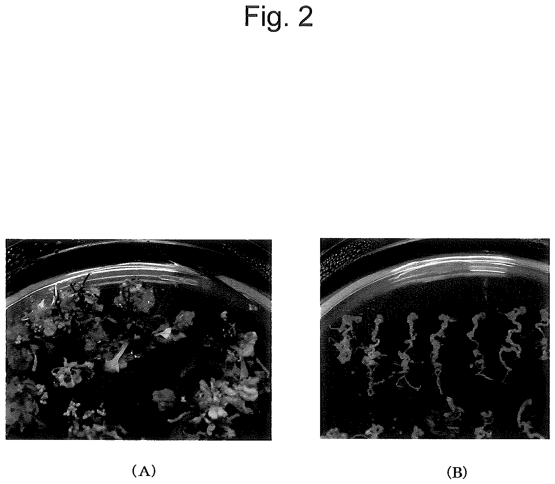

The root organ of *Arabidopsis (Columbia)* was cut, treated in a 1/2 MS medium supplemented with fipexide at 45 µM for 2 weeks to induce a callus, and then cultured in a redifferentiation medium (auxin/cytokinine). As a result, redifferentiation was observed (Fig. 2A).

In contrast, the root organ of *Arabidopsis (Columbia)* was cut and then cultured in the redifferentiation medium without callus induction. As a result, redifferentiation was not observed (Fig. 2B).

### (3) Comparison of fipexide with conventional technique

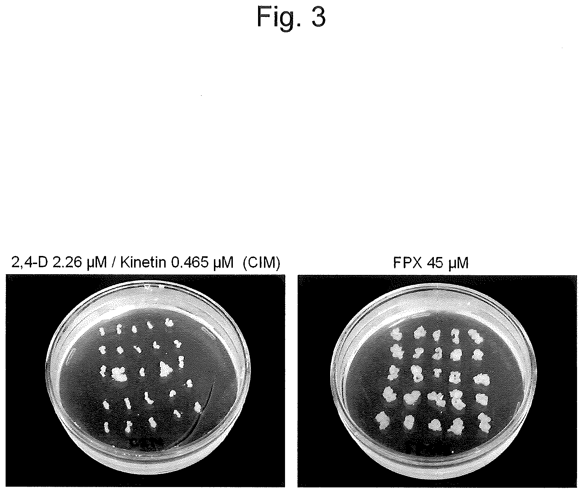

Callus induction in *Arabidopsis* that was carried out at the optimized auxin/cytokinine concentration (i.e., 2,4-dichlorophenoxyacetic acid at 2.26 µM and kinetin at 0.465 µM) was compared with callus induction that was carried out at the fipexide concentration of 45 µM. As a result, callus induction efficiency of the fipexide was found to be higher than that attained at the optimized auxin/cytokinine concentration. Fig. 3 shows the conditions of the hypocotyl that had been cultured under constant lighting conditions for 87 days.

### (4) Functional analysis of putative fipexide metabolites (i.e., 4-chlorophenoxyacetic acid and 1-piperonylpiperazine)

Fipexide is a compound formed by an amide bond between 4-chlorophenoxyacetic acid and 1-piperonylpiperazine, and fipexide is deduced to be non-enzymatically hydrolyzed in a plant. Thus, treatment with 4-chlorophenoxyacetic acid or 1-piperonylpiperazine alone or treatment with both thereof was attempted.

*Arabidopsis (Columbia)* was allowed to germinate under weak lighting conditions for 7 days, the hypocotyl cut therefrom was placed on a 1/2 MS medium supplemented with 4-chlorophenoxyacetic acid and/or 1-piperonylpiperazine at 0, 5, 15, 30, 45, or 60 µM, and morphological observation thereof was conducted.

As a result, callus induction activity was observed by treatment with 4-chlorophenoxyacetic acid as with the case of treatment with fipexide. This indicates that the 4-chlorophenoxyacetic acid structure plays a key role in callus formation.

When the plant was treated with 1-piperonylpiperazine alone, in contrast, the number and the length of lateral roots were increased, and the leaf area was enlarged, although callus induction activity was not observed.

When the hypocotyl of *Arabidopsis* was treated with both 4-chlorophenoxyacetic acid and 1-piperonylpiperazine, in addition, the callus growth rate was faster than the case in which the plant was treated with 4-chlorophenoxyacetic acid alone. This indicates that cell elongation activity of 1-piperonylpiperazine additively accelerates the callus induction caused by 4-chlorophenoxyacetic acid.

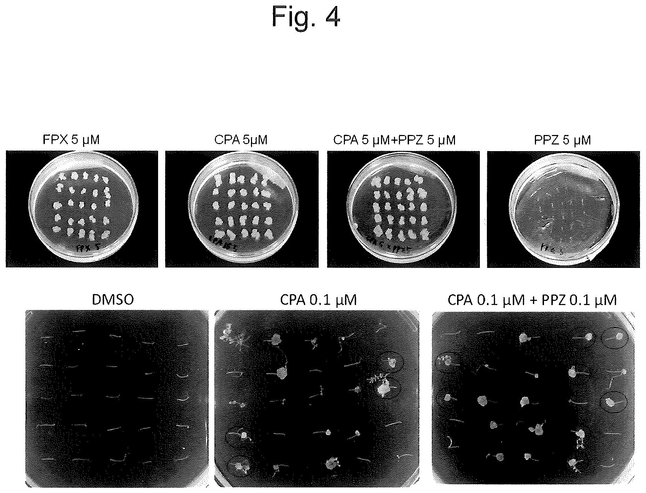

The results are shown in Fig. 4.

### [Example 2] Fipexide activity test using other plants

As with the case of *Arabidopsis* germination conditions, plants were cultured in a fipexide medium with the use of sterilized seeds. As a result, callus induction was observed in germinated rice seeds, germinated *Eucalyptus* seeds, soybean seed (Tsurunoko), tomato seed (Micro-Tom), and cucumber seed (Natsu Suzumi).

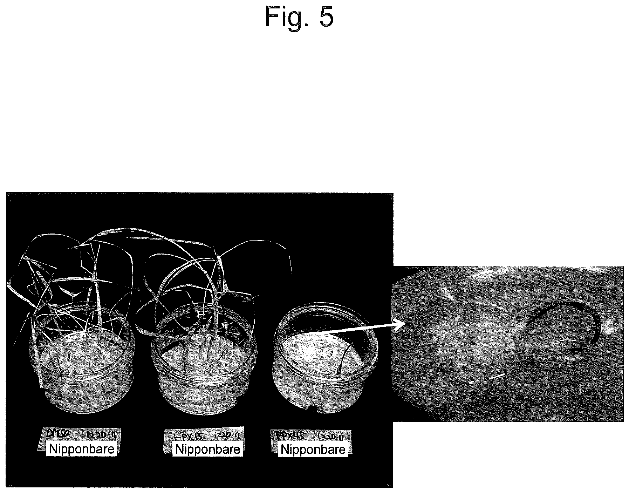

Fig. 5 shows the conditions of the wild-type rice (Nipponbare) 30 days after seed germination. Callus formation was observed at fipexide concentration of 45 µM.

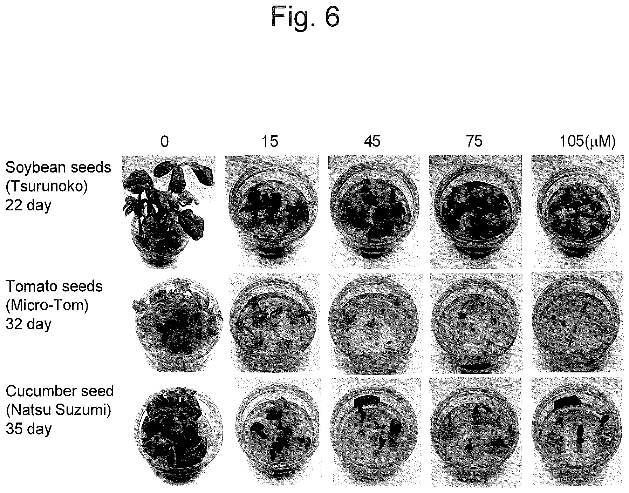

Fig. 6 shows the results of callus induction activity tests of fipexide (FPX) on soybean seed (Tsurunoko), tomato seed (Micro-Tom), and cucumber seed (Natsu Suzumi).

As with the case of *Arabidopsis* plants, stalks and roots of *Populus* were cultured in a fipexide medium and, as a result, callus induction was observed in the stalks and the roots of *Populus.*

The induced callus was cultured in a redifferentiation medium (i.e., a MS medium (0.9% agarose and 1.5% sucrose) containing auxin (indoleacetic acid) at 0.15 mg/l and cytokinine (N⁶-2-isopentenyladenine) at 0.5 mg/l or a 1/2 MS medium (0.9% Phytoagar (agarose for plants) and 0.2% sucrose) containing 3-indolebutyric acid (IBA) at 0.1 mg/l and 6-benzylaminopurine (BAP) at 0.2 mg/1). As a result, redifferentiation was observed.

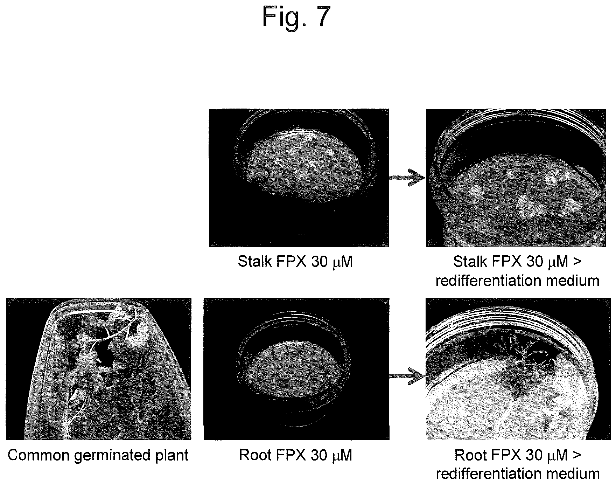

The conditions of the aseptic stalk (which is not the hypocotyl) and the root organ sections of wild-type *Populus* 30 days after cutting in the presence of fipexide are shown at the center of Fig. 7, and the conditions thereof 30 days after the medium was exchanged with a fresh redifferentiation medium are shown on the right of Fig. 7.

### [Example 3] Transformation utilizing callus induction caused by fipexide

Under general callus induction conditions for *Populus,* callus induction was conducted with the use of fipexide instead of auxin/cytokinine, and whether or not *Agrobacterium*-infected callus was transformed was examined.

The GUS gene was introduced into a pH35GS binary vector comprising a Gateway cassette (Kubo M. et al., Genes & Dev., 19, 1855-1860, 2005) (manufactured by Inplanta Innovations Inc.; Production code: IN3-VEC17) using the Gateway system, and the resulting binary vector was used for the *Agrobacterium* method, so as to confirm infection.

The transformation procedure is described below.
1. From young plants of aseptically cultured *Populus* (*Populus tremula* x *tremuloides* T89) that had been grown in pots, stalk samples (length: 5 mm) were cut.
2. The stalk samples were cultured in the presence of *Agrobacterium* (for 3 days, in the dark, at 22°C).
3. After *Agrobacterium* was washed away, the stalk samples were placed on MS1 medium or modified MS1 medium (containing 30 µM fipexide instead of 3-indolebutyric acid (IBA) and 6-benzylaminopurine (BAP)) and cultured at 25°C.
4. (On the fourth week, it was confirmed that many calluses were formed in modified MS1 medium containing fipexide while callus was not formed in conventional MS1 medium.)
5. Two months after the initiation of callus induction culture, 23 calluses were fixed in 90% (w/w) acetone at -30°C overnight, the samples were washed twice with 50 mM PBS buffer (pH 7.0), and the samples were subjected to incubation in GUS substrate solution at 37°C for 15 minutes for staining.

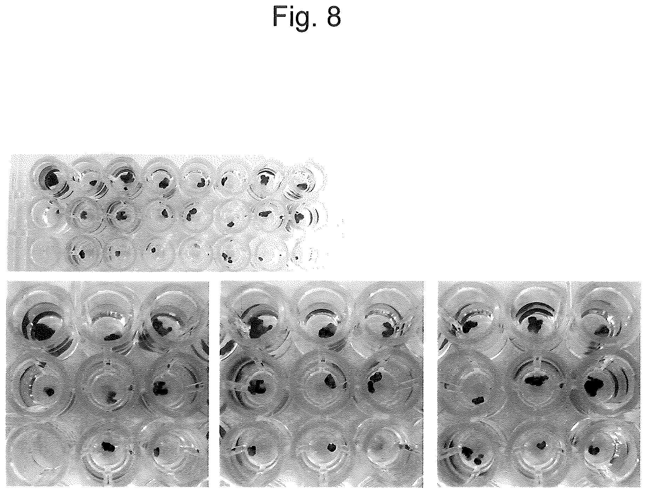

As a result, GUS activity was observed in all samples. The results are shown in Fig. 8.

The compositions of the MS1 medium, the modified MS1 medium, and the GUS substrate solution are shown below.

### [MS1 medium composition (in 1 liter)]

4.4 g of Murashige & Skoog salt
20 g of sucrose
0.2 mg of BAP
0.1 mg of IBA
0.01 mg of TDZ (thidiazuron)
pH 5.6
[Modified MS1 medium composition (in 1 liter)]
4.4 g of Murashige & Skoog salt
20 g of sucrose
30 µM of fipexide
pH 5.6
[GUS substrate solution composition (in 1 liter)]
1 mM of X-Gluc
50 mM of PBS (pH7.0)
0.1% of Triton X-100
1 mM of potassium ferricyanide
1 mM of potassium ferrocyanide

Thus, it was found that callus induction caused by fipexide was applicable to a plant transformation technique by the *Agrobacterium* method.

## Claims

1. Use of an agent for inducing a plant callus wherein the agent comprises a compound represented by Formula (I) or a salt thereof: 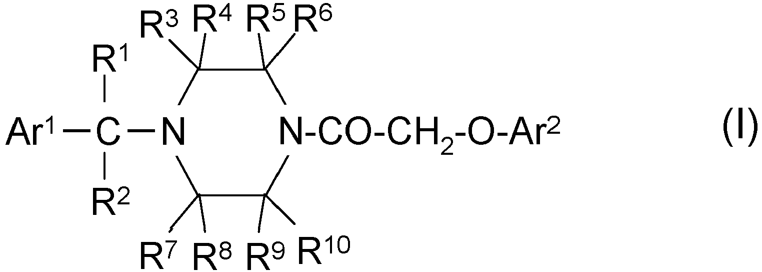
wherein Ar¹ represents a 3,4-methylenedioxyphenyl group;
Ar² represents a 4-chlorophenyl group;
R¹ and R² each represent a hydrogen atom; and
R³, R⁴, R⁵, R⁶, R⁷, R⁸, R⁹, and R¹⁰ each represent a hydrogen atom.

2. Use according to claim 1, wherein the compound represented by Formula (I) or a salt thereof is fipexide.

3. Use of an agent for inducing a plant callus wherein the agent comprises a compound represented by Formula (I-1) or a salt thereof: 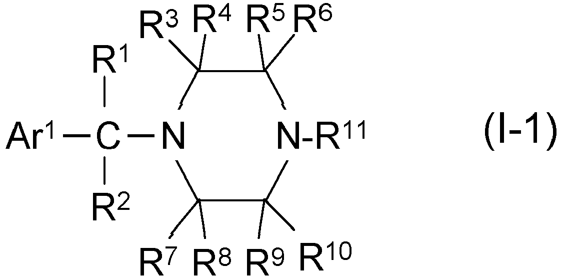 wherein Ar¹, R¹, R², R³, R⁴, R⁵, R⁶, R⁷, R⁸, R⁹, and R¹⁰ are as defined above with respect to Formula (I) in claim 1; and R¹¹ represents a hydrogen atom, and a compound represented by Formula (1-2) or a salt thereof:
HO-CO-CH₂-O-Ar² (I-2)
wherein Ar² is as defined above with respect to Formula (I) in claim 1.

4. Use according to claim 3, wherein the compound represented by Formula (I-1) or a salt thereof is 1-piperonylpiperazine and the compound represented by Formula (1-2) or a salt thereof is 4-chlorophenoxyacetic acid.

5. A method for inducing a plant callus comprising bringing a plant, a plant cell, a piece of plant tissue, or a plant seed into contact with the agent as defined in any one of claims 1 to 4 and inducing callus formation.

6. A method for producing a plant callus comprising bringing a plant, a plant cell, a piece of plant tissue, or a plant seed into contact with the agent as defined in any one of claims 1 to 4, inducing callus formation, and growing the callus.

7. A method according to claim 6, which further involves using the callus to produce a transformant.

## Patentansprüche

1. Verwendung eines Mittels zur Induktion eines Pflanzenkallus, wobei das Mittel eine Verbindung umfasst, die durch Formel (I) oder ein Salz davon dargestellt wird: 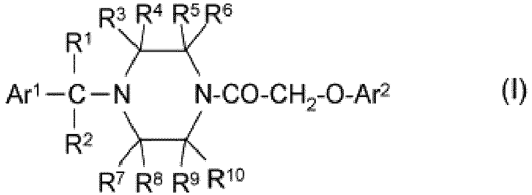
wobei Ar¹ eine 3,4-Methylendioxyphenylgruppe darstellt;
Ar² eine 4-Chlorphenylgruppe darstellt;
R¹ und R² jeweils ein Wasserstoffatom darstellen; und
R³, R⁴, R⁵, R⁶, R⁷, R⁸, R⁹ und R¹⁰ jeweils ein Wasserstoffatom darstellen.

2. Verwendung nach Anspruch 1, wobei die durch Formel (I) dargestellte Verbindung oder ein Salz davon Fipexid ist.

3. Verwendung eines Mittels zur Induktion eines Pflanzenkallus, wobei das Mittel eine Verbindung umfasst, die durch Formel (I-1) oder ein Salz davon dargestellt wird: 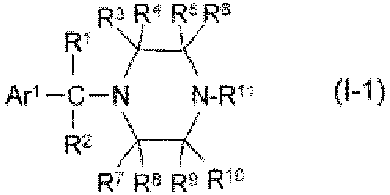 wobei Ar¹, R¹, R², R³, R⁴, R⁵, R⁶, R⁷, R⁸, R⁹ und R¹⁰ wie oben in Bezug auf Formel (I) in Anspruch 1 definiert sind; und R¹¹ ein Wasserstoffatom und eine Verbindung, die durch die Formel (I-2) oder ein Salz davon dargestellt wird, darstellen:
HO-CO-CH₂-O-Ar² (I-2)
wobei Ar² wie oben in Bezug auf Formel (I) in Anspruch 1 definiert ist.

4. Verwendung nach Anspruch 3, wobei die durch die Formel (I-1) dargestellte Verbindung oder ein Salz davon 1-Piperonylpiperazin ist und die durch die Formel (I-2) oder ein Salz davon dargestellte Verbindung 4-Chlorphenoxyessigsäure ist.

5. Verfahren zum Induzieren eines Pflanzenkallus, umfassend das Einbringen einer Pflanze, einer Pflanzenzelle, eines Stücks Pflanzengewebes oder eines Pflanzensamens in Kontakt mit dem Mittel, wie in einem der Ansprüche 1 bis 4 definiert und Induzieren der Kallusbildung.

6. Verfahren zum Herstellen eines Pflanzenkallus, umfassend das Einbringen einer Pflanze, einer Pflanzenzelle, eines Stücks Pflanzengewebes oder eines Pflanzensamens in Kontakt mit dem Mittel, wie in einem der Ansprüche 1 bis 4 definiert, Induzieren der Kallusbildung und Wachsen des Kallus.

7. Verfahren nach Anspruch 6, bei dem ferner der Kallus zur Herstellung einer Transformante verwendet wird.

## Revendications

1. Utilisation d'un agent pour provoquer un cal végétal, dans laquelle l'agent comprend un composé représentée par la formule (I) ou un sel de celui-ci : 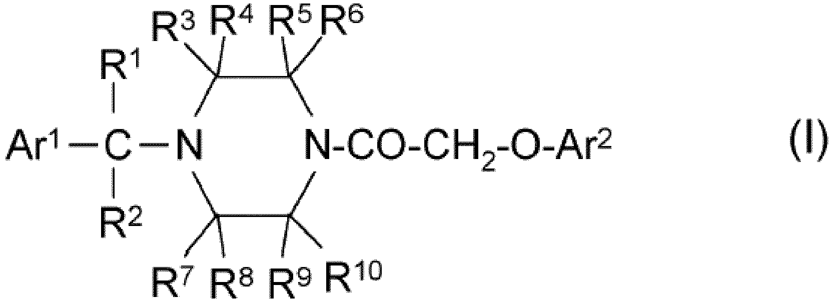
dans laquelle Ar¹ représente un groupe 3,4-méthylènedioxyphényle ;
Ar² représente un groupe 4-chlorophényle ;
R¹ et R² représentent chacun un atome d'hydrogène ; et
R³, R⁴, R⁵, R⁶, R⁷, R⁸, R⁹, et R¹⁰ représentent chacun un atome d'hydrogène.

2. Utilisation selon la revendication 1, dans laquelle le composé représenté par la formule (I) ou un sel de celui-ci est fipexide.

3. Utilisation d'un agent pour provoquer un cal végétal, dans laquelle l'agent comprend un composé représentée par la formule (I-1) ou un sel de celui-ci : 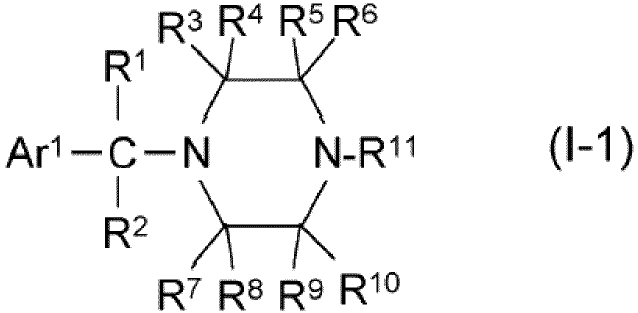 dans laquelle Ar¹, R¹, R², R³, R⁴, R⁵, R⁶, R⁷, R⁸, R⁹, et R¹⁰ sont tels que définis ci-dessus par rapport à la formule (I) dans la revendication 1 ; et R¹¹ représente un atome d'hydrogène, et un composé représentée par la formule (I-2) ou un sel de celui-ci :
HO-CO-CH₂-O-Ar² (1-2)
dans laquelle Ar² est tel que défini ci-dessus par rapport à la formule (I) dans la revendication 1.

4. Utilisation selon la revendication 3, dans laquelle le composé représenté par la formule (I-1) ou un sel de celui-ci est 1-pipéronylpipérazine et le composé représenté par la formule (I-2) ou un sel de celui-ci est acide 4-chlorophénoxyacétique.

5. Procédé pour provoquer un cal végétal, comprenant la mise d'une plante, d'une cellule végétale, d'un morceau de tissu végétal, ou d'une graine végétale en contact avec l'agent tel que défini dans l'une quelconque des revendications 1 à 4 et la provocation de la formation de cal.

6. Procédé pour produire un cal végétal, comprenant la mise d'une plante, d'une cellule végétale, d'un morceau de tissu végétal, ou d'une graine végétale en contact avec l'agent tel que défini dans l'une quelconque des revendications 1 à 4, la provocation de la formation de cal, et la croissance du cal.

7. Procédé selon la revendication 6, qui implique en outre l'utilisation du cal pour produire un transformé.
